# EUROPEAN PATENT SPECIFICATION

(11) **EP 1 869 172 B1**
(45) Date of publication and mention of the grant of the patent: **01.09.2010**
(21) Application number: 06798076.3
(22) Date of filing: 12.09.2006
(51) Int. Cl.: C12N 9/10, C07K 14/395

(54) **BRANCHED-CHAIN AMINO ACID AMINOTRANSFERASE GENE AND USE THEREOF**
GEN DER VERZWEIGTKETTIGEN AMINOSÄUREN-AMINOTRANSFERASE UND DESSEN VERWENDUNG
GENE D'AMINOTRANSFERASE D'ACIDE AMINE A CHAINE RAMIFIEE ET UTILISATION ASSOCIEE

(30) Priority: 13.09.2005 JP 2005266076; 26.12.2005 JP 2005371820
(43) Date of publication of application: 26.12.2007
(73) Proprietor: Suntory Holdings Limited, Kita-ku, Osaka-shi, Osaka 530-8203 (JP)
(72) Inventor: NAKAO, Yoshihiro, Mishima-gun, Osaka 618-8503 (JP); KODAMA, Yukiko, Mishima-gun, Osaka 618-8503 (JP); SHIMONAGA, Tomoko, Mishima-gun, Osaka 618-8503 (JP)
(74) Representative: Vossius & Partner
(86) International application number: PCT/JP2006/318465
(87) International publication number: WO 2007/032522

(56) References cited:
- WO-A-2004/057033
- WO-A2-99/50422
- KISPAL GYULA ET AL: "Mitochondrial and cytosolic branched-chain amino acid transaminases from yeast, homologs of the myc oncogene-regulated Eca39 protein" JOURNAL OF BIOLOGICAL CHEMISTRY, vol. 271, no. 40, 1996, pages 24458-24464, XP002418736 ISSN: 0021-9258

### SEQUENCE LISTING

<110> Suntory Limited
<120> Branched-chain amino acid aminotransferase gene and use thereof
<130> PCT06-0083
<150> JP 2005-266076
   <151> 2005-09-13
<150> JP 2005-371820
   <151> 2005-12-26
<160> 12
<170> PatentIn version 3.3
<210> 1
   <211> 1182
   <212> DNA
   <213> Saccharomyces sp.
<400> 1.
   atgttacaaa ggaattcttt gaaatggggg aaattctcca tcagaacgct agccactggt    60
   gctccattgg atgcctctaa attgaaaatt acaagaaacc caaatccttc caagccaaga   120
   ccaaatgacg aattagtgtt tggtcaaaca ttcacagacc acatgttgac cattccatgg   180
   actgccaaag aagggtgggg tgttcctcat atcaagcctt atgctaattt atcgcttgac   240
   ccatcatctt gtgtattcca ctatgcattt gaactgttcg aaggtttgaa agcctacaga   300
   acccctcaaa ataccattac catgttccgt tctgacaaga acatggttcg tatgaacaaa   360
   tccgcagcta gaatctgttt acccaccttc gactctgagg aattgatcaa gcttactggg   420
   aagttgatcg aacaagataa acatttagtt cctgaaggga atggttactc tttatacatc   480
   agaccaacaa tgatcggtac ctctaaaggt ttaggtgtcg gcactccttc cgaagctctt   540
   ctttacgtga tcacttctcc agttggtcct tattacaaga ccggtttcaa ggccgttcgt   600
   cttgaagcaa cagactatgc cactagagct tggccaggtg gtgttggtga caagaagttg   660
   ggtgctaact atgctccatg tatcttacct caattgcaag ctgctaaaag aggataccaa   720
   caaaacctat ggttgtttgg tcctgaaaaa aacattactg aagtcggcac catgaacgtg    780
   ttcttcgtct tcctcaacaa agtcaccggt aagaaagagt tggttactgc cccattagac    840
   ggtactatct tggaaggtgt cactagagat tctgttttga gtttggctcg tgagaaacta    900
   gatcctcaag aatgggacat caacgaacgt tattacacta tcactgaagt tgctactaga    960
   gctcaacaag gtgaactatt ggaagcattc ggttctggta ctgctgctgt tgtctcacca   1020
   atcaaggaaa ttggctggaa agatcaagat atcaatgttc cattattgcc tggtgagcaa   1080
   tctggtgctt tgaccaagca agtcgcccaa tggattgctg atattcaata cggtagaact   1140
   aagcacggta actggtcgag aactgttgct gacttgaact aa                       1182
<210> 2
   <211> 393
   <212> PRT
   <213> Saccharomyces sp.
<400> 2
   Met Leu Gln Arg Asn Ser Leu Lys Trp Gly Lys Phe Ser Ile Arg Thr
   1               5                   10                  15
   Leu Ala Thr Gly Ala Pro Leu Asp Ala Ser Lys Leu Lys Ile Thr Arg
               20                  25                  30
   Asn Pro Asn Pro Ser Lys Pro Arg Pro Asn Asp Glu Leu Val Phe Gly
           35                  40                  45
   Gln Thr Phe Thr Asp His Met Leu Thr Ile Pro Trp Thr Ala Lys Glu
       50                  55                  60
   Gly Trp Gly Val Pro His Ile Lys Pro Tyr Ala Asn Leu Ser Leu Asp
   65                  70                  75                  80
   Pro Ser Ser Cys Val Phe His Tyr Ala Phe Glu Leu Phe Glu Gly Leu
                   85                  90                  95
   Lys Ala Tyr Arg Thr Pro Gln Asn Thr Ile Thr Met Phe Arg Ser Asp
   100 105 110
   Lys Asn Met Val Arg Met Asn Lys Ser Ala Ala Arg Ile Cys Leu Pro
   115 120 125
   Thr Phe Asp Ser Glu Glu Leu Ile Lys Leu Thr Gly Lys Leu Ile Glu
   130 135 140
   Gln Asp Lys His Leu Val Pro Glu Gly Asn Gly Tyr Ser Leu Tyr Ile
   145 150 155 160
   Arg Pro Thr Met Ile Gly Thr Ser Lys Gly Leu Gly Val Gly Thr Pro
   165 170 175
   Ser Glu Ala Leu Leu Tyr Val Ile Thr Ser Pro Val Gly Pro Tyr Tyr
   180 185 190
   Lys Thr Gly Phe Lys Ala Val Arg Leu Glu Ala Thr Asp Tyr Ala Thr
   195 200 205
   Arg Ala Trp Pro Gly Gly Val Gly Asp Lys Lys Leu Gly Ala Asn Tyr
   210 215 220
   Ala Pro Cys Ile Leu Pro Gln Leu Gln Ala Ala Lys Arg Gly Tyr Gln
   225 230 235 240
   Gln Asn Leu Trp Leu Phe Gly Pro Glu Lys Asn Ile Thr Glu Val Gly
   245 250 255
   Thr Met Asn Val Phe Phe Val Phe Leu Asn Lys Val Thr Gly Lys Lys
   260 265 270
   Glu Leu Val Thr Ala Pro Leu Asp Gly Thr Ile Leu Glu Gly Val Thr
   275 280 285
   Arg Asp Ser Val Leu Ser Leu Ala Arg Glu Lys Leu Asp Pro Gln Glu
   290 295 300
   Trp Asp Ile Asn Glu Arg Tyr Tyr Thr Ile Thr Glu Val Ala Thr Arg
   305 310 315 320
   Ala Gln Gln Gly Glu Leu Leu Glu Ala Phe Gly Ser Gly Thr Ala Ala
   325 330 335
   Val Val Ser Pro Ile Lys Glu Ile Gly Trp Lys Asp Gln Asp Ile Asn
   340 345 350
   Val Pro Leu Leu Pro Gly Glu Gln Ser Gly Ala Leu Thr Lys Gln Val
   355 360 365
   Ala Gln Trp Ile Ala Asp Ile Gln Tyr Gly Arg Thr Lys His Gly Asn
   370 375 380
   Trp Ser Arg Thr Val Ala Asp Leu Asn
   385 390
<210> 3
   <211> 1131
   <212> DNA
   <213> Saccharomyces sp.
<400> 3
   atgaccttag cgcccttaga tgcctccaaa gtcaagatta ccaccaccga acacccatcg 60
   gagccaaaac caaacgacca gctggtcttt ggtagaagct tcaccgacca catgctaact 120
   gcggagtgga ctgcggcaaa cgggtgggac gtcccggaga tcaagcccta ccagaatctg 180
   tcactggacc catccgcgta cgtgttccac tatgcttttg agctgtttga aggtatgaag 240
   gcttacagga cagttgacaa taaaatcaca atgttccgcc cagacatgaa catgaagcgt 300
   atgaataagt ctgcccgacg aatttgtctg ccagctttcg actcggagga gttaattgcc 360
   ttgattgcaa agctgatcca acaagataag cgtttgattc cggagggaaa gggctactct 420
   ttgtacatca ggccaactat gattggcact acggccggcc tgggtgtctc cacgccagac 480
   agagccttgc tatatgtcat ttgttccccc gtgggccctt actacaagac cggtttcaaa 540
   gcggttaggc tggaagccac tgattacgcc acaagggctt ggccgggcgg ctgtggtgac 600
   aagaaattgg gcgccaacta tgctccttgc gtcctaccac agttgcaggc cgcctcaaga 660
   ggttaccaac agaacttgtg gttgtttggc ccagagaata atatcactga agtaggcacc 720
   atgaacgcat tctttgtctt caaaaacacc gaaacaggca agaaggaact ggtcactgcc 780
   ccattggacg gcaccattct ggaaggtgtc acgagagatt ccatattaac cctcgccaag 840
   gaaaggctcg atttaaatga gtggactatt agtgaacgct actgcaccat aggcgaagtt 900
   tctgagagat ccaaaaaagg cgaactagta gaggcttttg gttctggtac agccgcgatc 960
   gtttctccca tcaaagaaat cggctggaag ggcgaaccta tcaacatccc attactgccc 1020
   ggcgagcaaa cgggaccatt gaccaaggaa gttgcccaat ggattaacca aatccaatac 1080
   ggcgaagttg aacatggcaa ttggtcaagg gtcatcaccg atttgaactg a 1131
<210> 4
   <211> 376
   <212> PRT
   <213> Saccharomyces sp.
<400> 4
   Met Thr Leu Ala Pro Leu Asp Ala Ser Lys Val Lys Ile Thr Thr Thr
   1               5                   10                  15
   Glu His Pro Ser Glu Pro Lys Pro Asn Asp Gln Leu Val Phe Gly Arg
               20                  25                  30
   Ser Phe Thr Asp His Met Leu Thr Ala Glu Trp Thr Ala Ala Asn Gly
           35                  40                  45
   Trp Asp Val Pro Glu Ile Lys Pro Tyr Gln Asn Leu Ser Leu Asp Pro
   50 55 60
   Ser Ala Tyr Val Phe His Tyr Ala Phe Glu Leu Phe Glu Gly Met Lys
   65 70 75 80
   Ala Tyr Arg Thr Val Asp Asn Lys Ile Thr Met Phe Arg Pro Asp Met
   85 90 95
   Asn Met Lys Arg Met Asn Lys Ser Ala Arg Arg Ile Cys Leu Pro Ala
   100 105 110
   Phe Asp Ser Glu Glu Leu Ile Ala Leu Ile Ala Lys Leu Ile Gln Gln
   115 120 125
   Asp Lys Arg Leu Ile Pro Glu Gly Lys Gly Tyr Ser Leu Tyr Ile Arg
   130 135 140
   Pro Thr Met Ile Gly Thr Thr Ala Gly Leu Gly Val Ser Thr Pro Asp
   145 150 155 160
   Arg Ala Leu Leu Tyr Val Ile Cys Ser Pro Val Gly Pro Tyr Tyr Lys
   165 170 175
   Thr Gly Phe Lys Ala Val Arg Leu Glu Ala Thr Asp Tyr Ala Thr Arg
   180 185 190
   Ala Trp Pro Gly Gly Cys Gly Asp Lys Lys Leu Gly Ala Asn Tyr Ala
   195 200 205
   Pro Cys Val Leu Pro Gln Leu Gln Ala Ala Ser Arg Gly Tyr Gln Gln
   210 215 220
   Asn Leu Trp Leu Phe Gly Pro Glu Asn Asn Ile Thr Glu Val Gly Thr
   225 230 235 240
   Met Asn Ala Phe Phe Val Phe Lys Asn Thr Glu Thr Gly Lys Lys Glu
   245 250 255
   Leu Val Thr Ala Pro Leu Asp Gly Thr Ile Leu Glu Gly Val Thr Arg
   260 265 270
   Asp Ser Ile Leu Thr Leu Ala Lys Glu Arg Leu Asp Leu Asn Glu Trp
   275 280 285
   Thr Ile Ser Glu Arg Tyr Cys Thr Ile Gly Glu Val Ser Glu Arg Ser
   290 295 300
   Lys Lys Gly Glu Leu Val Glu Ala Phe Gly Ser Gly Thr Ala Ala Ile
   305 310 315 320
   Val Ser Pro Ile Lys Glu Ile Gly Trp Lys Gly Glu Pro Ile Asn Ile
   325 330 335
   Pro Leu Leu Pro Gly Glu Gln Thr Gly Pro Leu Thr Lys Glu Val Ala
   340 345 350
   Gln Trp Ile Asn Gln Ile Gln Tyr Gly Glu Val Glu His Gly Asn Trp
   355 360 365
   Ser Arg Val Ile Thr Asp Leu Asn
   370 375
<210> 5
   <211> 40
   <212> DNA
   <213> Artificial
<220>
   <223> nonScBATI_for
<400> 5
   gagctcatag cggccatgtt acaaaggaat tctttgaaat 40
<210> 6
   <211> 42
   <212> DNA
   <213> Artificial
<220>
   <223> nonScBATI_rv
<400> 6
   ggatcctatg cggccgcatg tgaattataa aatatgtatc ta 42
<210> 7
   <211> 40
   <212> DNA
   <213> Artificial
<220>
   <223> nonScBAT2_for
<400> 7
   gagctcatag cggccatgac cttagcgccc ttagatgcct 40
<210> 8
<211> 42
   <212> DNA
   <213> Artificial
<220>.
   <223> nonScBAT2_rv
<400> 8
   ggatcctatg cggccgctag aatatggaaa aaaaaatgct gc 42
<210> 9
   <211> 70
   <212> DNA
   <213> Artificial
<220>
   <223> nonScBAT1_delta_for
<400> 9
   ttaccctttt tctcctaagt acgtataagt tcagcccaac gtactaaaac ccttgacagt 60
   cttgacgtgc 70
<210> 10
   <211> 70
   <212> DNA
   <213> Artificial
<220>
   <223> nonScBAT1_delta_rv
<400> 10
   ttgttgttcg tttttttagc attagttcaa gtcagcaaca gttctcgacc cgcacttaac 60
   ttcgcatctg 70
<210> 11
   <211> 70
   <212> DNA.
   <213> Artificial
<220>
   <223> nonScBAT2_delta_for
<400> 11
   acatcagtaa catctttaca cacttaaaaa caacaacaac aacaacaaca ccttgacagt 60
   cttgacgtgc 70
<210> 12
   <211> 70
   <212> DNA
   <213> Artificial
<220>
   <223> nonScBAT2_delta_rv
<400> 12
   aaagagaatg cttttgtgtt ggctttttta tggtttgatg tagcagtact cgcacttaac 60
   ttcgcatctg 70

## Description

### TECHNICAL FIELD

The present invention relates to a branched-chain amino acid aminotransferase gene and a use thereof, in particular, a brewery yeast for producing alcoholic drinks with enhanced flavor, alcoholic drinks produced with said yeast and a method for producing said drinks. More particularly, the present invention relates to a yeast whose capacity of producing amyl alcohol and/or isobutanol and/or isoamyl acetate that contribute to the product flavor is controlled by controlling the expression level of BAT1 or BAT2 gene coding for brewery yeast branched-chain amino acid aminotransferase Bat1p or Bat2p, particularly nonScBAT1 or nonScBAT2 gene characteristic of beer yeast, and to a method for producing alcoholic drinks with said yeast.

### BACKGROUND ART

In alcoholic drinks, higher alcohols such as amyl alcohol and isobutanol and esters such as isoamyl acetate are part of the important aroma components. For sake, wine, whisky and other alcohol drinks, an increase in higher alcohol or ester is known to give rich aroma to the alcohol drink, resulting in high sensual evaluation. For beer, on the other hand, although higher alcohol and ester are important aroma components, excessive quantity of higher alcohol or ester is disfavored as organic solvent odor or ester odor. Therefore, it is important to appropriately control the amount of higher alcohol or ester produced according to the types of alcohols.

So far, enhanced ester-producing yeasts have been bred in order to increase ester content in alcohol. The following methods have been reported in order to efficiently separate the enhanced ester-producing yeast by (or by not) mutating yeast: a method for obtaining a strain that is resistant to an agent that inhibits a fatty acid synthase such as cerulenin and is capable of highly producing caproic acid as well as a strain that is resistant to a leucine analogue such as 5,5,5-trifluoro-DL-leucine and is capable of increasing amyl alcohol and isoamyl acetate (Japanese Laid-Open Patent Application No. 2002-253211); and a method for obtaining a strain which has been grown in a medium containing steroid having a pregnane skeleton with hydroxylation at position 3 (Japanese Laid-Open Patent Application No. 2002-191355).

On the other hand, following examples have been reported as methods for breeding an yeast utilizing a genetic engineering technique: an example where *Saccharomyces cerevisiae* alcohol acetyltransferase gene *ATF1* is highly expressed with a brewery yeast (Japanese Laid-Open Patent Application No.06-062849); an example where an expression of *ATF1* is repressed (Japanese Laid-Open Patent Application No.06-253826); and an example where a brewery yeast esterase gene *EST2* is disrupted to increase the ester content (Japanese Laid-Open Patent Application No.09-234077). As methods for appropriately controlling the amount of higher alcohol or ester production, an example of enhancing or repressing the expression of ScBAT1 or ScBAT2 gene coding for *Saccharomyces cerevisiae* branched-chain amino acid aminotransferase (Japanese Laid-Open Patent Application No.11-235176) is known.

### DISCLOSURE OF INVENTION

As described above, variants have been prepared in order to increase the higher alcohol or ester content in the products. However, their results were sometimes problematic for practical use because, for example, unexpected delay in the fermentation or unfavorable increase in the aroma component were caused by the yeast. Thus, there has been a need for a method for breeding yeast capable of producing abundant amount of higher alcohol or ester without impairing the fermentation rates or quality of the products.

As a result of keen investigation by the present inventors for solving the above problems, they succeeded in identifying and isolating a gene coding for branched-chain amino acid aminotransferase from beer yeast which has advantageous effects than the existing proteins. Moreover, an yeast was transformed by introducing and expressing with the obtained gene to confirm that the amounts of higher alcohol and ester produced were increased, and an yeast was transformed such that the expression of the obtained gene was repressed to confirm that the amounts of higher alcohol and ester produced were decreased, thereby completing the present invention.

Thus, the present invention relates to a novel branched-chain amino acid aminotransferase gene as defined in the claims existing specifically in a beer yeast, to a protein coded by said gene, to a transformed yeast in which the expression of said gene is controlled, to a method for controlling the amount of amyl alcohol and/or isobutanol and/or isoamyl acetate in a product by using an yeast in which the expression of said gene is controlled. Specifically, the present invention provides the following polynucleotides, a vector containing said polynucleotide, a transformed yeast introduced with said vector, and a method for producing alcoholic drinks by using said transformed yeast.
[1] A polynucleotide selected from the group consisting of the following polynucleotides (a) to (d):
   (a) a polynucleotide comprising a polynucleotide consisting of the nucleotide sequence represented by SEQ ID NO:1 or SEQ ID NO:3;
   (b) a polynucleotide comprising a polynucleotide coding for a protein consisting of the amino acid sequence represented by SEQ ID NO:2 or SEQ ID NO:4;
   (c) a polynucleotide comprising a polynucleotide coding for a protein consisting of the amino acid sequence represented by SEQ ID NO:2 or SEQ ID NO:4 with one to 18 amino acids thereof being deleted, substituted, inserted and/or added, and having a branched-chain amino acid aminotransferase activity; and
   (d) a polynucleotide comprising a polynucleotide coding for a protein of an amino acid sequence having 95% or higher identity with the amino acid sequence represented by SEQ ID NO:2 or SEQ ID NO:4, and having a branched-chain amino acid aminotransferase activity.
[2] A polynucleotide according to [1] above, selected from the group consisting of the following polynucleotides (g) to (h):
   (g) a polynucleotide comprising a polynucleotide coding for a protein consisting of the amino acid sequence represented by SEQ ID NO:2 or SEQ ID NO:4 or of an amino acid sequence represented by SEQ ID NO:2 or SEQ ID NO:4 with 1 to 5 amino acids thereof being deleted, substituted, inserted and/or added, and having a branched-chain amino acid aminotransferase activity; and
   (h) a polynucleotide comprising a polynucleotide coding for a protein having an amino acid sequence having 99% or higher identity with the amino acid sequence represented by SEQ ID NO:2 or SEQ ID NO:4, and having a branched-chain amino acid aminotransferase activity.
[3] A polynucleotide according to [1] above, comprising a polynucleotide of the nucleotide sequence represented by SEQ ID NO:1 or SEQ ID NO:3.
[4] A polynucleotide according to [1] above, comprising a polynucleotide coding for a protein of the amino acid sequence represented by SEQ ID NO:2 or SEQ ID NO:4.
[5] A polynucleotide according to any one of [1] to [4] above, wherein the polynucleotide is DNA.
[6] A protein coded by the polynucleotide according to any one of [1] to [5] above.
[7] A vector comprising the polynucleotide according to any one of [1] to [5] above.
[7a] Also disclosed is a vector according to [7] above including expression cassette comprising the following components (x) to (z):
   (x) a promoter that can be transcribed in an yeast cell;
   (y) a polynucleotide according to any one of [1] to [5] above, linked to the promoter in a sense or antisense direction; and
   (z) a signal that functions in the yeast with respect to transcription termination and polyadenylation of RNA molecule.
[8] A yeast introduced with the vector according to [7] above.
[9] A yeast according to [8] above, having an enhanced capacity of producing amyl alcohol and/or isobutanol and/or isoamyl acetate by being introduced with the vector according to [7] above.
[10] A yeast in which the expression of the polynucleotide (DNA) according to [5] above is repressed by disrupting the gene related to the polynucleotide (DNA) according to [5] above.
[11] A yeast according to [9] above, having an enhanced capacity of producing amyl alcohol and/or isobutanol and/or isoamyl acetate by increasing the expression level of the protein according to [6] above.
[12] A method for producing an alcoholic drink comprising using the yeast according to any one of [8] to [11] above.
[13] A method for producing an alcoholic drink according to [12] above, wherein the brewed alcoholic drink is a malt drink.
[14] A method for producing an alcoholic drink according to [12] above, wherein the brewed alcoholic drink is wine.
[15] A method for assessing a test yeast for its capacity of producing amyl alcohol and/or isobutanol and/or isoamyl acetate, comprising using a primer or a probe comprising at least 15 nucleotides of a nucleotide sequence of a branched-chain amino acid aminotransferase gene having the nucleotide sequence represented by SEQ ID NO:1 or SEQ ID NO:3.
[15a] Also disclosed is a method for selecting an yeast with a higher or lower capacity of producing amyl alcohol and/or isobutanol and/or isoamyl acetate by the method according to [15] above.
[15b] Also disclosed is a method for producing an alcohol (e.g., beer) by using the yeast selected by the method according to [15a] above.
[16] A method for assessing a test yeast for its capacity of producing amyl alcohol and/or isobutanol and/or isoamyl acetate, comprising: culturing a test yeast; and determining an expression level of a branched-chain amino acid aminotransferase gene having the nucleotide sequence represented by SEQ ID NO: 1 or SEQ ID NO:3.
[17] A method for selecting a yeast; comprising: culturing test yeasts; quantifying the protein according to [6] above or determining an expression level of a branched-chain amino acid aminotransferase gene having the nucleotide sequence represented by SEQ ID NO:1 or SEQ ID NO:3; and selecting a test yeast having said protein amount or said gene expression level according to the capacity of producing target amyl alcohol and/or isobutanol and/or isoamyl acetate.
[17a] Also disclosed is a method for selecting an yeast, comprising: culturing test yeasts; and determining capacities of producing amyl alcohol and/or isobutanol and/or isoamyl acetate or a branched-chain amino acid aminotransferase activity; and selecting a test yeast having the intended capacity of producing amyl alcohol and/or isobutanol and/or isoamyl acetate or the intended branched-chain amino acid aminotransferase activity.
[18] A method for selecting a yeast according to [17] above, comprising: culturing a reference yeast and test yeasts; determining an expression level of a branched-chain amino acid aminotransferase gene having the nucleotide sequence represented by SEQ ID NO:1 or SEQ ID NO:3 in each yeast; and selecting a test yeast having the gene expressed higher or lower than that in the reference yeast.
[19] A method for selecting a yeast according to [18] above, comprising: culturing a reference yeast and test yeasts; quantifying the protein according to [6] above in each yeast; and selecting a test yeast having said protein for a larger or smaller amount than that in the reference yeast. Specifically, a method for selecting an yeast according to [18] above, comprising: culturing a plurality of yeasts; quantifying the protein according to [6] above in each of the yeasts; and selecting a test yeast among these yeast that has produced the protein for the larger or smaller amount.
[20] A method for producing an alcoholic drink comprising: conducting fermentation for producing an alcoholic drink using the yeast according to any one of [8] to [11] above or an yeast selected by the method according to any one of [17] to [19] above; and adjusting the production amount of amyl alcohol and/or isobutanol and/or isoamyl acetate.

According to the method for producing alcohols by using the transformed yeast of the invention, higher alcohol and ester contents can be controlled so that alcohols with enhanced flavor can be produced.

### BRIEF DESCRIPTION OF DRAWINGS

Figure 1 shows the change in the amount of yeast growth with time upon beer fermentation test. The horizontal axis represents fermentation time while the vertical axis represents OD660.
Figure 2 shows the change in the amount of extract consumption with time upon beer fermentation test. The horizontal axis represents fermentation time while the vertical axis represents apparent extract concentration (w/w%).
Figure 3 shows behavior of nonScBAT1 gene expression in yeast during beer fermentation test. The horizontal axis represents fermentation time while the vertical axis represents detected signal brightness.
Figure 4 shows the change in the amount of yeast growth with time upon beer fermentation test The horizontal axis represents fermentation time while the vertical axis represents OD660. The symbol "bat1" denotes a nonScBAT1 disrupted strain.
Figure 5 shows the change in the amount of extract consumption with time upon beer fermentation test. The horizontal axis represents fermentation time while the vertical axis represents apparent extract concentration (w/w%). The symbol "bat1" denotes a nonScBAT1 disrupted strain.
Figure 6 shows the change in the amount of yeast growth with time upon beer fermentation test. The horizontal axis represents fermentation time while the vertical axis represents OD660. The symbol "BAT1" denotes a nonScBAT1 highly expressed strain.
Figure 7 shows the change in the amount of extract consumption with time upon beer fermentation test. The horizontal axis represents fermentation time while the vertical axis represents apparent extract concentration (w/w%). The symbol "BAT1" denotes a nonScBAT1 highly expressed strain.
Figure. 8 shows the change in the amount of yeast growth with time upon beer fermentation test. The horizontal axis represents fermentation time while the vertical axis represents OD660.
Figure 9 shows the change in the amount of extract consumption with time upon beer fermentation test. The horizontal axis represents fermentation time while the vertical axis represents apparent extract concentration (w/w%).
Figure 10 shows behavior of nonScBAT2 gene expression in yeast during beer fermentation test. The horizontal axis represents fermentation time while the vertical axis represents detected signal brightness.
Figure 11 shows the change in the amount of yeast growth with time upon beer fermentation test. The horizontal axis represents fermentation time while the vertical axis represents OD660. The symbol "bat2" denotes a nonScBAT2 disrupted strain.
Figure 12 shows the change in the amount of extract consumption with time upon beer fermentation test. The horizontal axis represents fermentation time while the vertical axis represents apparent extract concentration (w/w%). The symbol "bat2" denotes a nonScBAT2 disrupted strain.
Figure 13 shows the change in the amount of yeast growth with time upon beer fermentation test. The horizontal axis represents fermentation time while the vertical axis represents OD660. The symbol "BAT2" denotes a nonScBAT2 highly expressed strain.
Figure 14 shows the change in the amount of extract consumption with time upon beer fermentation test. The horizontal axis represents fermentation time while the vertical axis represents apparent extract concentration (w/w%). The symbol "BAT2" denotes a nonScBAT2 highly expressed strain.

### BEST MODES FOR CARRYING OUT THE INVENTION

The present inventors thought that the amounts of higher alcohol and ester can be controlled by increasing or decreasing the branched-chain amino acid aminotransferase activity of an yeast. They have studied based on this idea and as a result, isolated and identified nonScBAT1 gene and nonScBAT2 gene coding for branched-chain amino acid aminotransferases unique to beer yeast based on the beer yeast genome information mapped according to the method disclosed in Japanese Laid-Open Patent Application No. 2004-283169. These nucleotide sequences are represented by SEQ ID NO:1 and SEQ ID NO:3, respectively. Amino acid sequences of proteins coded by these genes are represented by SEQ ID NO:2 and SEQ ID NO:4, respectively.

### 1. Polynucleotide of the invention

First of all, the present invention provides (a) a polynucleotides comprising a polynucleotide of the nucleotide sequence represented by SEQ ID NO:1 or SEQ ID NO:3; and (b) a polynucleotide comprising a polynucleotide coding for a protein of the amino acid sequence represented by SEQ ID NO:2 or SEQ ID NO:4. The polynucleotide can be DNA or RNA. The target polynucleotide of the present invention is not limited to the polynucleotide coding for a branched-chain amino acid aminotransferase gene from beer yeast and may include other polynucleotide coding for proteins having equivalent functions to said protein. Proteins with equivalent functions include (c) a protein of an amino acid sequence represented by SEQ ID NO:2 or SEQ ID NO:4 with one or more amino acids thereof being deleted, substituted, inserted and/or added and having a branched-chain amino acid aminotransferase activity. Such proteins include a protein consisting of an amino acid sequence represented by SEQ ID NO:2 or SEQ ID NO:4 with, for example, 1 to 18, 1 to 17, 1 to 16, 1 to 15, 1 to 14, 1 to 13, 1 to 12, 1 to 11, 1 to 10, 1 to 9, 1 to 8, 1 to 7, 1 to 6 (1 to several amino acids), 1 to 5, 1 to 4, 1 to 3, 1 to 2, or 1 amino acid residues thereof being deleted, substituted, inserted and/or added and having a branched-chain amino acid aminotransferase activity. In general, the number of deletion, substitution, insertion and/or addition is preferably smaller. In addition, such proteins include (d) a protein having an amino acid sequence with 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher identity with the amino acid sequence represented by SEQ ID NO:2 or SEQ ID NO:4, and having a branched-chain amino acid aminotransferase activity. In general, the percentage identity is preferably higher.

The branched-chain amino acid aminotransferase activity may be determined, for example, by the method described in J. Biol, Chem., 271, 2445 8-24464, 1996.

Furthermore, the present invention discloses a polynucleotide comprising a polynucleotide which hybridizes to a polynucleotide consisting of a nucleotide sequence complementary to the nucleotide sequence represented by SEQ ID NO:1 or SEQ ID NO:3 under stringent conditions and which codes for a protein having a branched-chain amino acid aminotransferase activity; and a polynucleotide comprising a polynucleotide which hybridizes to a polynucleotide consisting of a nucleotide sequence complementary to a nucleotide sequence of a polynucleotide coding for a protein of the amino acid sequence represented by SEQ ID NO:2 or SEQ ID NO:4 under stringent conditions and which codes for a protein having a branched-chain amino acid aminotransferase activity.

Herein, "a polynucleotide that hybridizes under stringent conditions" refers to a polynucleotide, such as a DNA, obtained by a colony hybridization technique, a plaque hybridization technique, a southern hybridization technique or the like using all or part of a polynucleotide of a nucleotide sequence complementary to the nucleotide sequence represented by SEQ ID NO:1 or SEQ ID NO:3 or a polynucleotide coding for the amino acid sequence represented by SEQ ID NO:2 or SEQ ID NO:4 as a probe. The hybridization method may be a method described, for example, in Molecular Cloning 3rd Ed., Current Protocols in Molecular Biology, John Wiley & Sons 1987-1997.

The term "stringent conditions" as used herein may be any of low stringent conditions, moderate stringent conditions or high stringent conditions. "Low stringent conditions" are, for example, 5 x SSC, 5 x Denhardt's solution, 0.5% SDS, 50% formamide at 32°C. "Moderate stringent conditions" are, for example, 5 x SSC, 5 x Denhardt's solution, 0.5% SDS, 50% formamide at 42°C. "High stringent conditions" are, for example, 5 x SSC, 5 x Denhardt's solution, 0.5% SDS, 50% formamide at 50°C. Under these conditions, a polynucleotide, such as a DNA, with higher homology is expected to be obtained efficiently at higher temperature, although multiple factors are involved in hybridization stringency including temperature, probe concentration, probe length, ionic strength, time, salt concentration and others, and one skilled in the art may appropriately select these factors to realize similar stringency.

When a commercially available kit is used for hybridization, for example, Alkphos Direct Labelling Reagents (Amersham Pharmacia) may be used. In this case, according to the attached protocol, after incubation with a labeled probe overnight, the membrane is washed with a primary wash buffer containing 0.1% (w/v) SDS at 55°C, thereby detecting a hybridized polynucleotide such as a DNA.

Other polynucleotides that can be hybridized include polynucleotides having about 60% or higher, about 70% or higher, 71% or higher, 72% or higher, 73% or higher, 74% or higher, 75% or higher, 76% or higher, 77% or higher, 78% or higher, 79% or higher, 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher or 99.9% or higher identity to the polynucleotide coding for the amino acid sequence represented by SEQ ID NO:2 or SEQ ID NO:4 as calculated by a homology search software such as FASTA and BLAST using default parameters.

Identity between amino acid sequences or nucleotide sequences may be determined using algorithm BLAST by Karlin and Altschul (Proc. Natl. Acad. Sci. USA, 87, 2264-2268, 1990; Proc. Natl. Acad. Sci. USA, 90, 5873, 1993). Programs called BLASTN and BLASTX based on BLAST algorithm have been developed (Altschul SF, et al: J. Mol. Biol. 215: 403, 1990). When a nucleotide sequence is sequenced using BLASTN, parameters are, for example, score = 100 and wordlength = 12. When an amino acid sequence is sequenced using BLASTX, parameters are, for example, score = 50 and wordlength = 3. When BLAST and Gapped BLAST programs are used, default parameters for each of the programs are employed.

Also disclosed is (j) a polynucleotide coding for RNA having a nucleotide sequence complementary to a transcript of the polynucleotide (DNA) according to (5) above; (k) a polynucleotide coding for RNA that represses the expression of the polynucleotide (DNA) according to (5) above through RNAi effect; (1) a polynucleotide coding for RNA having an activity of specifically cleaving a transcript of the polynucleotide (DNA) according to (5) above; and (m) a polynucleotide coding for RNA that represses expression of the polynucleotide (DNA) according to (5) above through co-repression effect. These polynucleotides may be incorporated into a vector, which can be introduced into a cell for transformation to repress the expression of the polynucleotides (DNA) of (a) to (i) above. Thus, these polynucleotides may suitably be used when repression of the expression of the above polynucleotide such as a DNA is preferable.

The phrase "polynucleotide coding for RNA having a nucleotide sequence complementary to the transcript of DNA" as used herein refers to so-called antisense DNA. Antisense technique is known as a method for repressing expression of a particular endogenous gene, and is described in various publications (see e.g., Hirajima and Inoue: New Biochemistry Experiment Course 2 Nucleic Acids IV Gene Replication and Expression (Japanese Biochemical Society Ed., Tokyo Kagaku Dozin Co., Ltd.) pp.319-347, 1993). The sequence of antisense DNA is preferably complementary to all or part of the endogenous gene, but may not be completely complementary as long as it can effectively repress the expression of the gene. The transcribed RNA has preferably 90% or higher, and more preferably 95% or higher complementarity to the transcript of the target gene. The length of the antisense DNA is at least 15 bases or more, preferably 100 bases or more, and more preferably 500 bases or more.

The phrase "polynucleotide coding for RNA that represses DNA expression through RNAi effect" as used herein refers to a polynucleotide for repressing expression of an endogenous gene through RNA interference (RNAi). The term "RNAi" refers to a phenomenon where when double-stranded RNA having a sequence identical or similar to the target gene sequence is introduced into a cell, the expressions of both the introduced foreign gene and the target endogenous gene are repressed. RNA as used herein includes, for example, double-stranded RNA that causes RNA interference of 21 to 25 base length, for example, dsRNA (double strand RNA), siRNA (small interfering RNA) or shRNA (short hairpin RNA). Such RNA may be locally delivered to a desired site with a delivery system such as liposome, or a vector that generates the double-stranded RNA described above may be used for local expression thereof. Methods for producing or using such double-stranded RNA (dsRNA, siRNA or shRNA) are known from many publications (see, e.g., Japanese National Phase PCT Laid-open Patent Publication No. 2002-516062; US 2002/086356A; Nature Genetics, 24(2), 180-183, 2000 Feb.; Genesis, 26(4), 240-244, 2000 April; Nature, 407:6802, 319-20, 2002 Sep. 21; Genes & Dev., Vol.16, (8), 948-958, 2002 Apr. 15; Proc. Natl. Acad. Sci. USA., 99(8), 5515-5520, 2002 Apr. 16; Science, 296(5567), 550-553, 2002 Apr. 19; Proc Natl. Acad. Sci. USA, 99:9, 6047-6052, 2002 Apr. 30; Nature Biotechnology, Vol.20 (5), 497-500, 2002 May; Nature Biotechnology, Vol. 20(5), 500-505,2002 May; Nucleic Acids Res., 30:10, e46,2002 May 15).

The phrase "polynucleotide coding for RNA having an activity of specifically cleaving transcript of DNA" as used herein generally refers to a ribozyme. Ribozyme is an RNA molecule with a catalytic activity that cleaves a transcript of a target DNA and inhibits the function of that gene. Design of ribozymes can be found in various known publications (see, e.g., FEBS Lett. 228: 228, 1988; FEBS Lett. 239: 285, 1988; Nucl. Acids. Res. 17: 7059, 1989; Nature 323: 349, 1986; Nucl. Acids. Res. 19: 6751, 1991; Protein Eng 3: 733, 1990; Nucl. Acids Res. 19: 3875, 1991; Nucl. Acids Res. 19: 5125, 1991; Biochem Biophys Res Commun 186: 1271, 1992). In addition, the phrase "polynucleotide coding for RNA that represses DNA expression through co-repression effect" refers to a nucleotide that inhibits functions of target DNA by "co-repression".

The term "co-repression" as used herein, refers to a phenomenon where when a gene having a sequence identical or similar to a target endogenous gene is transformed into a cell, the expressions of both the introduced foreign gene and the target endogenous gene are repressed. Design of polynucleotides having a co-repression effect can also be found in various publications

(see, e.g., Smyth DR: Curr. Biol. 7: R793, 1997, Martienssen R: Curr. Biol. 6: 810, 1996).

### 2. Protein of the present invention

The present invention also provides proteins coded by any of the polynucleotides (a) to (h) above. A preferred protein of the present invention comprises an amino acid sequence represented by SEQ ID NO:2 or SEQ ID NO:4.

Such protein includes those having an amino acid sequence represented by SEQ ID NO:2 or SEQ ID NO:4 with amino acid residues thereof of the number mentioned above being deleted, substituted, inserted and/or added and having a branched-chain amino acid aminotransferase activity. In addition, such protein includes those having homology as described above with the amino acid sequence represented by SEQ ID NO:2 or SEQ ID NO:4 and having a branched-chain amino acid aminotransferase activity.

Such proteins may be obtained by employing site-directed mutation described, for example, in "Molecular Cloning 3rd Ed.", "Current Protocols in Molecular Biology", "Nuc. Acids. Res., 10, 6487 (1982)", "Proc. Natl. Acad. Sci. USA, 79, 6409 (1982)", "Gene, 34, 315 (1985)", "Nuc. Acids. Res.,13, 4431 (1985)", "Proc. Natl. Acad. Sci. USA, 82,488 (1985)".

Deletion, substitution, insertion and/or addition of one or more amino acid residues in an amino acid sequence of the protein of the invention means that the above mentioned number of amino acid residues is deleted, substituted, inserted and/or added at any one or more positions in the same amino acid sequence. Two or more types of deletion, substitution, insertion and/or addition may occur concurrently.

Hereinafter, examples of mutually substitutable amino acid residues are enumerated.

Amino acid residues in the same group are mutually substitutable. Group A: leucine, isoleucine, norleucine, valine, norvaline, alanine, 2-aminobutanoic acid, methionine, o-methylserine, t-butylglycine, t-butylalanine, cyclohexylalanine; Group B: asparatic acid, glutamic acid, isoasparatic acid, isoglutamic acid, 2-aminoadipic acid, 2-aminosuberic acid; Group C: asparagine, glutamine; Group D: lysine, arginine, "ornithine, 2,4-diaminobutanoic acid, 2,3-diaminopropionic acid; Group E: proline, 3-hydroxyproline, 4-hydroxyproline; Group F: serine, threonine, homoserine; and Group G: phenylalanine, tyrosine.

The protein of the present invention may also be produced by chemical synthesis methods such as Fmoc method (fluorenylmethyloxycarbonyl method) and tBoc method (t-butyloxycarbonyl method). In addition, peptide synthesizers available from, for example, Advanced ChemTech, PerkinElmer, Pharmacia, Protein Technology Instrument, Synthecell-Vega,

PerSeptive, Shimazu Corp. can also be used for chemical synthesis.

### 3. Vector of the invention and yeast transformed with the vector

The present invention then provides a vector comprising the polynucleotide described above. The vector of the present invention includes any of the polynucleotides (e.g., DNA) described in (a) to (h). Generally, the vector of the present invention comprises an expression cassette including as components (x) a promoter that can transcribe in a yeast cell; (y) a polynucleotide (DNA) described in any of (a) to (h) above that is linked to the promoter in sense or antisense direction; and (z) a signal that functions in the yeast with respect to transcription termination and polyadenylation of RNA molecule. According to the present invention, in order to highly express the protein of the invention described above upon brewing alcoholic drinks (e.g., beer) described below, these polynucleotides are introduced into the promoter in the sense direction to promote expression of the polynucleotide (DNA) described in any of (a) to (h) above. Also disclosed is the possibility to repress the expression of the polynucleotide of the present invention. In order to repress the expression of the above protein of the invention upon brewing alcoholic drinks (e.g., beer) as described below, the polynucleotide can be introduced into the promoter in the antisense direction to repress the expression of the polynucleotide (DNA) described in any of (a) to (h). In order to repress the above protein of the invention, the polynucleotide may be introduced such that the polynucleotide of any of the (j) to (m) is expressed. According to the present invention, the target gene (DNA) may be disrupted to repress the expression of the polynucleotide (e.g., DNA) or the protein. A gene may be disrupted by adding or deleting one or more bases to or from a region involved in expression of the gene product in the target gene, for example, a coding region or a promoter region, or by deleting these regions entirely. Such disruption of gene may be found in known publications (see, e.g., Proc. Natl. Acad. Sci. USA, 76, 4951(1979), Methods in Enzymology, 101, 202(1983), Japanese Laid-Open Patent Application No.6-253826).

A vector introduced in the yeast may be any of a multicopy type (YEp type), a single copy type (YCp type), or a chromosome integration type (YIp type). For example, YEp24 (J. R. Broach et al., Experimental Manipulation of Gene Expression, Academic Press, New York, 83, 1983) is known as a YEp type vector, YCp50 (M. D. Rose et al., gene, 60, 237, 1987) is known as a YCp type vector, and YIp5 (K. Struhl et al., Proc. Natl. Acad. Sci. USA, 76, 1035, 1979) is known as a YIp type vector, all of which are readily available.

Promoters/terminators for adjusting gene expression in yeast may be in any combination as long as they function in the brewery yeast and they have no influence on the constituents, such as an amino acid, sugar, a higher alcohol or an ester in fermentation broth. For example, a promoter of glyceraldehydes 3-phosphate dehydrogenase gene (TDH3), or a promoter of 3-phosphoglycerate kinase gene (PGK1) may be used. These genes have previously been cloned, described in detail, for example, in M. F. Tuite et al., EMBO J., 1, 603 (1982), and are readily available by known methods.

Since an auxotrophy marker cannot be used as a selective marker upon transformation for a brewery yeast, for example, a geneticin-resistant gene (G418r), a copper-resistant gene (CUP1) (Marin et al., Proc. Natl. Acad. Sci. USA, 81, 337 1984) or a cerulenin-resistant gene (fas2m, PDR4) (Junji Inokoshi et al., Biochemistry, 64, 660, 1992; and Hussain et al., gene, 101, 149, 1991, respectively) may be used. A vector constructed as described above is introduced into a host yeast Examples of the host yeast include any yeast that can be used for brewing, for example, brewery yeasts for beer, wine and sake. Specifically, yeasts such as genus *Saccharomyces* may be used. According to the present invention, a beer yeast, for example, *Saccharomyces pastorianus* W34/70, *Saccharomyces carlsbergensis* NCYC453 or NCYC456, or *Saccharomyces cerevisiae* NBRC1951, NBRC1952, NBRC1953 or NBRC1954 may be used. In addition, whisky yeasts such as *Saccharomyces cerevisiae* NCYC90, wine yeasts such as wine yeasts #1, 3 and 4 from the Brewing Society of Japan, and sake yeasts such as sake yeast #7 and 9 from the Brewing Society of Japan may also be used but not limited thereto. In the present invention, beer yeasts such as *Saccharomyces pastorianus* may be used preferably.

An yeast transformation method may be a generally used known method. For example, methods that can be used include but not limited to an electroporation method (Meth. Enzym., 194, p182 (1990)), a spheroplast method (Proc. Natl. Acad. Sci. USA, 75 p1929(1978)), a lithium acetate method (J. Bacteriology, 153, p163(1983)), and methods described in Proc. Natl. Acad. Sci. USA, 75 p1929 (1978), Methods in yeast genetics, 2000 Edition : A Cold Spring Harbor Laboratory Course Manual.

More specifically, a host yeast is cultured in a standard yeast nutrition medium (e.g., YEPD medium (Genetic Engineering. Vol.1, Plenum Press, New York, 117(1979)), etc.) such that OD600nm will be 1 to 6. This culture yeast is collected by centrifugation, washed and pre-treated with alkali metal ion, preferably lithium ion at a concentration of about 1 to 2M. After the cell is left to stand at about 30°C for about 60 minutes, it is left to stand with DNA to be introduced (about 1 to 20 µg) at about 30°C for about another 60 minutes. Polyethyleneglycol, preferably about 4,000 dalton of polyethyleneglycol, is added to a final concentration of about 20% to 50%. After leaving at about 30°C for about 30 minutes, the cell is heated at about 42°C for about 5 minutes. Preferably, this cell suspension is washed with a standard yeast nutrition medium, added to a predetermined amount of fresh standard yeast nutrition medium and left to stand at about 30°C for about 60 minutes. Thereafter, it is seeded to a standard agar medium containing an antibiotic or the like as a selective marker to obtain a transformant. Other general cloning techniques may be found, for example, in "Molecular Cloning 3rd Ed. ", "Methods in Yeast Genetics, A laboratory manual (Cold Spring Harbor Laboratory Press, Cold Spring Harbor, NY)".

### 4. Method of producing alcoholic drinks according to the present invention and alcoholic drinks produced by the method

The vector of the present invention described above is introduced into a yeast suitable for brewing a target alcoholic product. This yeast can be used to produce a desired alcoholic drink with enhanced flavor with an increased content of amyl alcohol and/or isobutanol and/or isoamyl acetate. In addition, yeasts to be selected by the yeast assessment method of the present invention described below can also be used. The target alcoholic drinks include, for example, but not limited to beer, sparkling liquor such as a beer-taste drink, wine, whisky and sake. According to the present invention, if needed, a brewery yeast in which an expression of a target gene is repressed may be used to produce a desired alcoholic drink with a decreased content of amyl alcohol and/or isobutanol and/or isoamyl acetate. Thus, an yeast introduced with the vector of the invention described above, an yeast in which the expression of the polynucleotide (DNA) of the invention described above is repressed, or an yeast selected by an yeast assessment method of the invention described below can be used for fermentation for producing alcoholic drinks and adjusting (increasing or decreasing) the amount of amyl alcohol and/or isobutanol and/or isoamyl acetate produced, thereby producing desired alcoholic drinks with adjusted (increased or decreased) content of amyl alcohol and/or isobutanol and/or isoamyl acetate.

In order to produce these alcoholic drinks, a known technique can be used except that a brewery yeast obtained according to the present invention is used in the place of a parent strain. Since materials, manufacturing equipment, manufacturing control and the like may be exactly the same as the conventional ones, there is no need of increasing the cost for producing alcoholic drinks with an controlled content of amyl alcohol and/or isobutanol and/or isoamyl acetate. Thus, according to the present invention, alcoholic drinks with enhanced flavor can be produced using the existing facility without increasing the cost.

### 5. Assessment method for yeast of the invention

The present invention relates to a method for assessing a test yeast for its capacity of producing amyl alcohol and/or isobutanol and/or isoamyl acetate by using a primer or a probe comprising at least 15 nucleotides of a nucleotide sequence of a branched-chain amino acid aminotransferase gene having the nucleotide sequence represented by SEQ ID NO:1 or SEQ ID NO:3. General techniques for such assessment method is known and is described in, for example, WO01/040514, Japanese Laid-Open Patent Application No.8-205900 or the like. This assessment method will be described in brief below.

First, genome of a test yeast is prepared. For this preparation, any known method such as Hereford method or potassium acetate method may be used (e.g., Methods in Yeast Genetics, Cold Spring Harbor Laboratory Press, p130 (1990)). Using a primer or a probe, as mentioned above, designed based on a nucleotide sequence (preferably, ORF sequence) of the branched-chain amino acid aminotransferase gene, the existence of the gene or a sequence specific to the gene is determined in the obtained test yeast genome. The primer or the probe may be designed according to a known technique.

Detection of the gene or the specific sequence may be carried out by employing a known technique. For example, a polynucleotide including part or all of the specific sequence or a polynucleotide including a nucleotide sequence complementary to said nucleotide sequence is used as one primer while a polynucleotide including part or all of the sequence upstream or downstream from this sequence or a polynucleotide including a nucleotide sequence complementary to said nucleotide sequence is used as another primer to amplify a nucleic acid of the yeast by a PCR method, thereby determining the existence of amplified products and molecular weight of the amplified products. The number of bases of polynucleotide used for a primer is generally 10 bp or more, and preferably 15 to 25 bp. In general, the number of bases between the primers is suitably 300 to 2000 bp.

The reaction conditions for PCR are not particularly limited but may be, for example, a denaturation temperature of 90 to 95°C, an annealing temperature of 40 to 60°C, an elongation temperature of 60 to 75°C, and the number of cycle of 10 or more. The resulting reaction product may be separated, for example, by electrophoresis using agarose gel to determine the molecular weight of the amplified product. This method allows prediction and assessment of the capacity of the yeast to produce amyl alcohol and/or isobutanol and/or isoamyl acetate as determined by whether the molecular weight of the amplified product is a size that contains the DNA molecule of the specific part. In addition, by analyzing the nucleotide sequence of the amplified product, the capacity may be predicted and/or assessed more precisely.

Moreover, in the present invention, a test yeast is cultured to determine an expression level of the branched-chain amino acid aminotransferase gene having the nucleotide sequence represented by SEQ ID NO:1 or SEQ ID NO:3 to assess the test yeast for its capacity of producing amyl alcohol and/or isobutanol and/or isoamyl acetate. In this case, the test yeast is cultured and then mRNA or a protein resulting from the branched-chain amino acid aminotransferase gene is quantified. The quantification of mRNA or protein may be carried out by employing a known technique. For example, mRNA may be quantified, by Northern hybridization or quantitative RT-PCR, while protein may be quantified, for example, by Western blotting (Current Protocols in Molecular Biology, John Wiley & Sons 1994-2003).

Furthermore, test yeasts are cultured and expression levels of the branched-chain amino acid aminotransferase gene having the nucleotide sequence represented by SEQ ID NO:1 or SEQ ID NO:3 are determined to select a test yeast with the gene expression level according to the capacity of producing target amyl alcohol and/or isobutanol and/or isoamyl acetate, thereby selecting a yeast favorable for brewing desired alcoholic drinks. In addition, a reference yeast and a test yeast may be cultured so as to determine and compare the expression level of the gene in each of the yeasts, thereby selecting favorable test yeast. Specifically, for example, a reference yeast and test yeasts are cultured and an expression level of the branched-chain amino acid aminotransferase gene having the nucleotide sequence represented by SEQ ID NO: or SEQ ID NO:3 is determined in each yeast. By selecting a test yeast with the gene expressed higher than that in the reference yeast, an yeast suitable for brewing alcoholic drinks can be selected.

Alternatively, test yeasts are cultured and an yeast with a higher or lower capacity of producing amyl alcohol and/or isobutanol and/or isoamyl acetate, or an yeast with a higher or lower branched-chain amino acid aminotransferase activity is selected, thereby selecting an yeast suitable for brewing desired alcoholic drinks.

In these cases, the test yeasts or the reference yeast may be, for example, an yeast introduced with the vector of the invention, an yeast in which the expression of the polynucleotide (DNA) of the invention described above has been controlled, an artificially mutated yeast or a naturally mutated yeast The capacity of producing amyl alcohol and/or isobutanol and/or isoamyl acetate may be determined by employing head-space gas chromatography method (J. Am. Soc. Brew. Chem.49:152-157,1991), while a branched-chain amino acid aminotransferase activity may be determined according to a method described in J. Biol, Chem., 271, 24458-24464, 1996. The mutation treatment may employ any methods including, for example, physical methods such as ultraviolet irradiation and radiation irradiation, and chemical methods associated with treatments with drugs such as EMS (ethylmethane sulphonate) and N-methyl-N-nitrosoguanidine (see, e.g.,Yasuji Oshima Ed., Biochemistry Experiments vol.39, Yeast molecular genetic experiments, pp.67-75, JSSP).

Examples of yeasts used as the reference yeast or the test yeasts include any yeasts that can be used for brewing, for example, brewery yeasts for beer, wine and sake. Specifically, yeasts such as genus *Saccharomyces* may be used (*e.g., S. pastorianus, S. cerevisiae,* and *S. carlsbergensis*). According to the present invention, a beer yeast, for example, *Saccharomyces pastorianus* W34/70, *Saccharomyces carlsbergensis* NCYC453 or NCYC456, or *Saccharomyces cerevisiae* NBRC 1951, NBRC1952, NBRC 1953 or NBRC1954 may be used. In addition, wine yeasts such as wine yeasts #1, 3 and 4 from the Brewing Society of Japan, and sake yeasts such as sake yeast #7 and 9 from the Brewing Society of Japan may also be used but not limited thereto. In the present invention, beer yeasts such as *Saccharomyces pastorianus* may be used preferably. The reference yeast and the test yeasts may be selected from the above yeasts in any combination.

### EXAMPLES

Hereinafter, the present invention will be described in more detail by way of examples. The present invention, however, is not limited to these examples below.

### Example 1: Cloning of novel branched-chain amino acid aminotransferase genre (nonScBAT1)

We found a novel branched-chain amino acid aminotransferase gene, nonScBAT1 (SEQ ID NO:1), unique to a beer yeast, as a result of a search utilizing the comparison database described in Japanese Laid-Open Patent Application No. 2004-283169. Based on the acquired nucleotide sequence information, primers nonScBAT1_for (SEQ ID NO:5) and nonScBAT1_rv (SEQ ID NO:6) were designed to amplify a full-length gene. PCR was carried out using chromosomal DNA of a genome sequencing strain, *Saccharomyces pastorianus* Weihenstephan 34/70 strain, also abbreviated to "W34/70 strain", as a template to obtain a DNA fragment (about 1.2kb) including the full-length gene of nonScBAT1.

The thus-obtained nonScBAT1 gene fragment was inserted into pCR2.1-TOPO vector (Invitrogen) by TA cloning. The nucleotide sequence of nonScBAT1 gene was analyzed according to Sanger's method (F. Sanger, Science, 214, 1215, 1981) to confirm the nucleotide sequence.

### Example 2: Analysis of nonScBAT1 gene expression during beer fermentation

A beer fermentation test was conducted using a lager brewing yeast, *Saccharomyces pastorianus* Weihenstephan 34/70 strain and then mRNA extracted from yeast cells during fermentation was analyzed by a beer yeast DNA microarray.

| | |
|---|---|
| Wort extract concentration | 12.69% |
| Wort content | 70L |
| Wort dissolved oxygen concentration | 8.6ppm |
| Fermentation temperature | 15°C |
| Yeast pitching rate | 12.8 x 10⁶cells/mL |

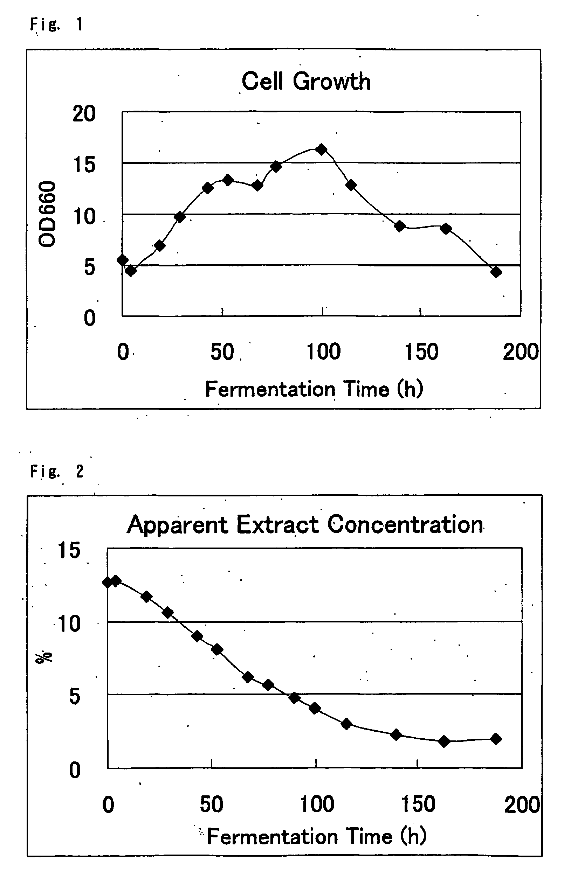

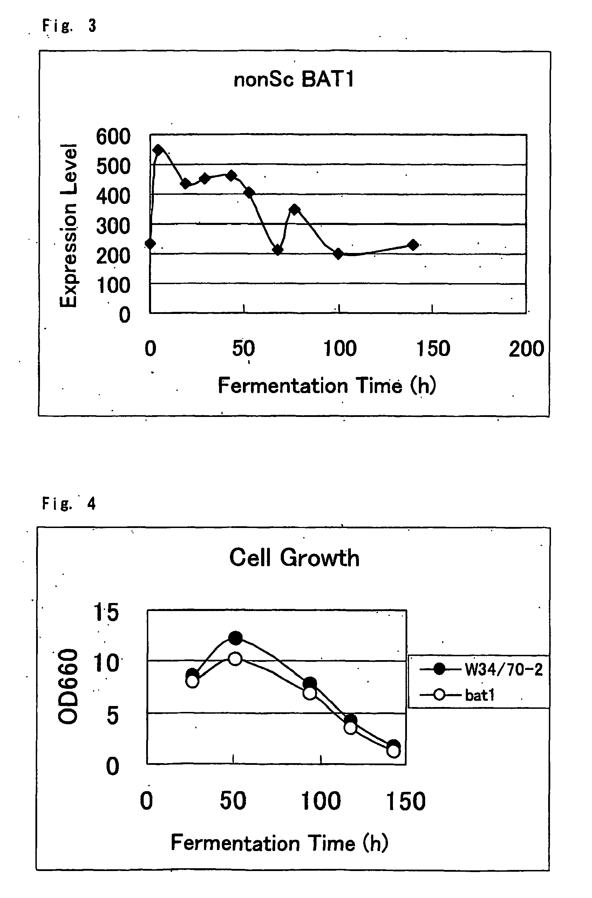

The fermentation broth was sampled with time to observe the amount of yeast growth (Figure 1) and apparent extract concentration (Figure 2) with time. At the same time, the yeast strain wars sampled, from which mRNA was prepared, biotin labeled, and hybridized to a lager brewing yeast DNA microarray. The signal was detected using a GeneChip Operating System (GCOS;GeneChip Operating Software 1.0, Affymetrix). An expression pattern of nonScBAT1 gene is shown in Figure 3. From this result, nonScBAT1 gene was confirmed to be expressed upon usual beer fermentation.

### Example 3: Disruption of nonScBAT1 gene

According to the publication (Goldstein et aL, yeast. 15 1541 (1999)), PCR using a plasmid including a drug-resistant marker (pFA6a (G418r)) as a template was conducted to prepare a fragment for gene disruption. As PCR primers, nonScBAT1_delta_for (SEQ ID NO:9) and nonScBAT1_delta_rv (SEQ ID NO: 10) were used.

The fragment for gene disruption prepared by the method above was used to transform a spore cloning strain (W34/70-2) separated from beer yeast *Saccharomyces pastorianus* W34/70 strain. Transformation was carried out as described in Japanese Laid-Open Patent Application No.07-303475, and transformants were selected in a YPD plate medium (1% yeast extract, 2% polypeptone, 2% glucose, 2% agar) containing 300mg/L Geneticin.

### Example 4: Analysis of amounts of amyl alcohol, isobutanol and isoamyl acetate produced upon beer fermentation test

Fermentation tests using a parent strain and the nonScBAT1 disrupted strain obtained in Example 3 were conducted under the following conditions.

| | |
|---|---|
| Wort extract concentration | 12% |
| Wort content | 1L |
| Wort dissolved oxygen concentration | 10ppm |
| Fermentation temperature | 15°C |
| Yeast pitching rate | 5g/L |

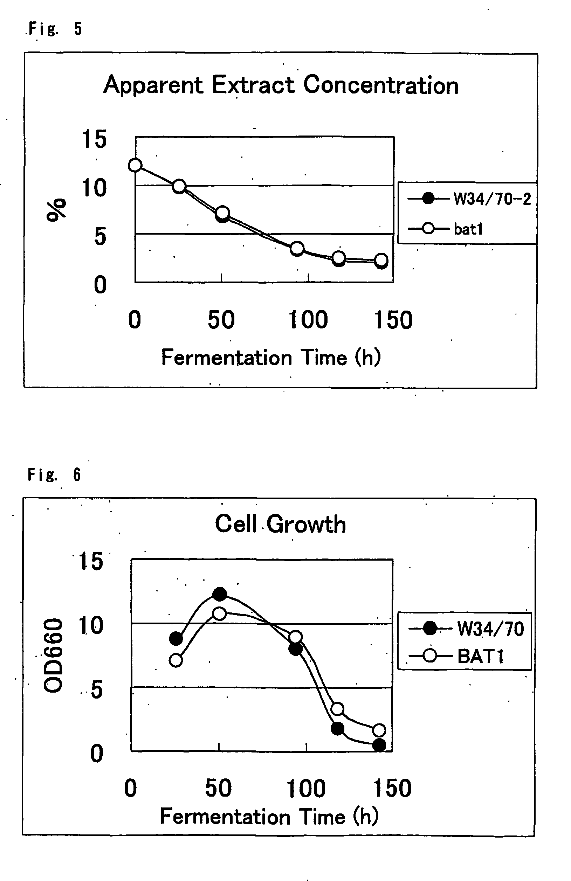

The fermentation broth was sampled with time to observe the amount of yeast growth (OD660) (Figure 4) and the amount of extract consumption (Figure 5). Quantifications of amyl alcohol, isobutanol and isoamyl acetate concentrations at the end of the fermentation were conducted using head-space gas chromatography (J. Am. Soc. Brew. Chem.49:152-157,1991).

Referring to Table 1, the amounts of isobutanol production at the end of fermentation were 22.3ppm in the parent strain and 14.9ppm in the nonScBAT1 disrupted strain. The amounts of amyl alcohol production were 95.2ppm in the parent strain and 77.6ppm in the nonScBAT1 disrupted strain. The amounts of isoamyl acetate production were 3ppm in the parent strain and 1.8ppm in the nonScBAT1 disrupted strain. From these results, it was revealed that nonScBAT1 disruption decreased the amounts of isobutanol, amyl alcohol and isoamyl acetate production by about 20-40%. In these cases, differences in the growth rates and the extract consumption rates were little between the parent strain and the disrupted strain.

**Table 1**

| | Parent strain (W34/70-2) | nonScBAT1 disrupted strain | |
|---|---|---|---|
| Isobutanol | 22.3 | 14.9 | (66.8%) |
| Amyl alcohol | 95.2 | 77.6 | (81.5%) |
| Isoamyl acetate | 3 | 1.8 | (60%) |

| | | | |
|---|---|---|---|
| Unit: ppm. Values in parentheses indicate percentages relative to the parent strain. | | | |

### Example 5: Preparation of nonScBAT1 highly expressed strain

nonScBAT1/pCR2.1-TOPO described in Example 1 was digested with restriction enzymes SacI and NotI to prepare a DNA fragment including the entire protein coding region. This fragment was linked to pYCGPYNot treated with restriction enzymes SacI and NotI, thereby , constructing a nonScBAT1 high expression vector, nonScBAT1/pYCGPYNot. pYCGPYNot was a YCp type yeast expression vector, and the introduced gene was highly expressed by the promoter of pyruvate kinase gene PYK1. Geneticin-resistant gene G418^{r} as a selective marker for yeast and ampicillin-resistant gene Amp^{r} as a selective marker for *E.coli* were also included ,

The high expression vector prepared by the method above was used to transform *Saccharomyces pastorianus* Weihenstephan 34/70 strain according to the method described in Japanese Laid-Open Patent Application No.07-303475. The transformants were selected in a YPD plate medium (1% yeast extract, 2% polypeptone, 2% glucose, 2% agar) containing 300mg/L geneticin.

### Example 6: Analysis of amounts of amyl alcohol, isobutanol and isoamyl acetate produced upon beer fermentation test

Fermentation tests using the parent strain and the nonScBAT1 highly expressed strain obtained in Example 5 were carried out under the following conditions.

| | |
|---|---|
| Wort extract concentration | 12% |
| Wort content | 1L |
| Wort dissolved oxygen concentration | 10ppm |
| Fermentation temperature | 15°C |
| Yeast pitching rate | 5g/L |

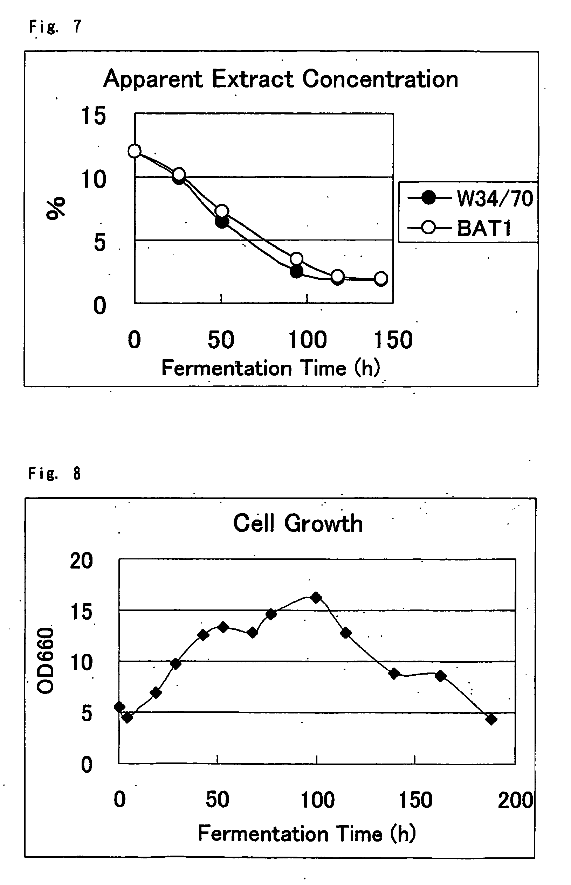

The fermentation broth was sampled with time to observe the changes in the amount of yeast growth (OD660) (Figure 6) and the amount of extract consumption (Figure 7) with time. Quantifications of higher alcohol and ester concentrations at the end of fermentation were conducted using head-space gas chromatography (J. Am. Soc. Brew. Chem.49:152-157,1991).

Referring to Table 2, the amounts of isobutanol production at the end of fermentation were 11.1ppm in the parent strain and 12.6ppm in the nonScBAT1 highly expressed strain. The amounts of amyl alcohol production were 61ppm in the parent strain and 65.7ppm in the nonScBAT1 highly expressed strain. The amounts of isoamyl acetate production were 1.8ppm in the parent strain and 2ppm in the nonScBAT1 highly expressed strain. From these results, it was revealed that the amounts of isobutanol, amyl alcohol and isoamyl acetate produced were increased by about 1.1 times by nonScBAT1 high expression. In these cases, differences in the growth rates and the extract consumption rates were little between the parent strain and the highly expressed strain.

**Table 2**

| | Parent strain (W34/70) | nonScBAT1 highly expressed strain | |
|---|---|---|---|
| Isobutanol | 11.1 | 12.6 | (114%) |
| Amyl alcohol | 61 | 65.7 | (108%) |
| Isoamyl acetate | 1.8 | 2 | (109%) |

| | | | |
|---|---|---|---|
| Unit: ppm Values in parentheses indicate percentages relative to the parent strain. | | | |

### Example 7: Cloning of novel branched-chain amino acid aminotransferase (nonScBAT2) gene

As a result of searching using the comparison database described in Japanese Laid-Open Patent Application No. 2004-283169, a novel branched-chain amino acid aminotransferase gene unique to beer yeast, nonScBAT2, was found (SEQ ID NO:3). Based on the obtained nucleotide sequence information, primers nonScBAT2_for (SEQ ID NO:7) and nonScBAT2_rv (SEQ ID NO:8) were designed to amplify the full-length gene. A DNA fragment (about 1.2kb) including a full-length gene of nonScBAT2 was obtained by PCR using chromosomal DNA of a genome sequencing strain, *Saccharomyces pastorianus* Weihenstephan 34/70 strain as a template.

The nonScBAT2 gene fragment obtained as described above was inserted into pCR2.1-TOPO vector (Invitrogen) by TA cloning. The nucleotide sequence of nonScBAT22 gene was analyzed by Sanger's method (F. Sanger, Science, 214, 1215, 1981) to confirm the nucleotide sequence.

### Example 8: Analysis of nonScBAT2 gene expression during beer fermentation test

A beer fermentation test was conducted using a lager brewing yeast, *Saccharomyces pastorianus* Weihenstephan 34/70 strain and then mRNA extracted from yeast cells during fermentation was analyzed by a beer yeast DNA microarray.

| | |
|---|---|
| Wort extract concentration | 12.69% |
| Wort content | 70L |
| Wort dissolved oxygen concentration | 8.6ppm |
| Fermentation temperature | 15°C |
| Yeast pitching rate | 12.8 x 10⁶cells/mL |

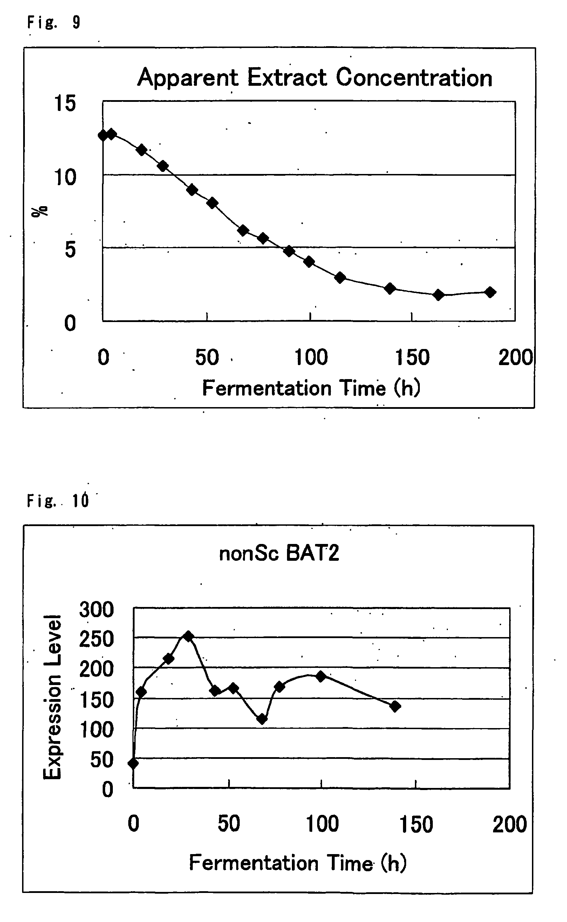

The fermentation broth was sampled with time to observe changes in the amounts of yeast growth (Figure 8) and apparent extract concentrations (Figure 9) with time. At the same time, yeast strains were sampled, from which mRNA was prepared, biotin labeled, and hybridized to a lager brewing yeast DNA microarray. Detection of signals were conducted using GeneChip Operating System (GCOS;GeneChip Operating Software 1.0, Affymetrix). The expression pattern of nonScBAT2 gene is shown in Figure 10. From these results, it was confirmed that nonScBAT2 gene was expressed in usual beer fermentation.

### Example 9: Disruption of nonScBAT2 gene

According to the publication (Goldstein et aL, yeast. 15 1541 (1999)), PCR using a plasmid including drug-resistant markers (pFA6a (G418r), pAG25 (nat1) and pAG32 (hph)) as a template was carried out to prepare a fragment for gene disruption. As PCR primers, nonScBAT2_delta_for (SEQ ID NO:11) and nonScBAT2_delta_rv (SEQ ID NO:12) were used

With the fragment for gene disruption prepared in the method described above, beer yeast *Saccharomyces pastorianus* W34/70 strain or a spore cloning strain (W34/70-2) separated from W34/70 was transformed. The transformation took place as described in Japanese Laid-Open Patent Application No.07-303475, and a transformant was selected in a YPD plate medium (1% yeast extract, 2% polypeptone, 2% glucose, 2% agar) containing 300mg/L Geneticin, 50mg/L Nourseothricin or 200mg/L Hygromycin B.

### Example 10: Analysis of amounts of amyl alcohol isobutanol and isoamyl acetate produced upon beer fermentation test

Fermentation tests using a parent strain and the nonScBAT2 disrupted strain obtained ins Example 9 were conducted under the following conditions.

| | |
|---|---|
| Wort extract concentration | 11.85% |
| Wort content | 1L |
| Wort dissolved oxygen concentration | 8ppm |
| Fermentation temperature | 15°C |
| Yeastpitching rate | 5g/L |

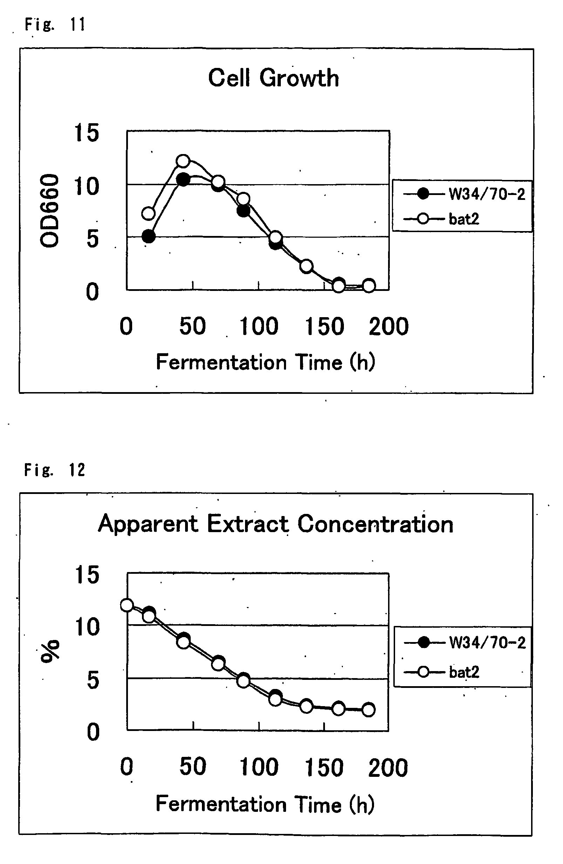

The fermentation broth was sampled with time to observe the changes in the amounts of yeast growth (OD660) (Figure 11) and the amounts of extract consumption (Figure 12) with time. Quantifications of higher alcohol and ester concentrations at the end of fermentation were carried out by employing head-space gas chromatography (J. Am. Soc. Brew. Chem. 49:152-157, 1991).

Referring to Table 3, the amounts of isobutanol produced at the end of fermentation were 18.5ppm in the parent strain and 7.3ppm in the nonScBAT2 disrupted strain. The amounts of amyl alcohol produced were 86.2ppm in the parent strain and 50.8ppm in the nonScBAT2 disrupted strain. The amounts of isoamyl acetate produced were 2.3ppm in the parent strain and 1.3ppm in the nonScBAT2 disrupted strain. From these results, it was revealed that nonScBAT2 disruption decreased the amounts of isobutanol, amyl alcohol and isoamyl acetate produced by about 40 to 60%. In these cases, differences in the growth rates and the extract consumption rates were little between the parent strain and the disrupted strain.

**Table 3**

| | Parent strain (W34/70-2) | nonScBAT2disrupted strain | |
|---|---|---|---|
| Isobutanol | 18.5 | 7.3 | (39.5%) |
| Amyl alcohol | 86.2 | 50.8 | (58.9%) |
| Isoamyl acetate | 2.3 | 1.3 | (56.5%) |

| | | | |
|---|---|---|---|
| Unit: ppm. Values in parentheses indicate percentages relative to the parent strain. | | | |

### Example 11: Preparation of nonScBAT2 highly expressed strain

nonScBAT2/pCR2.1-TOPO described in Example 7 was digested with restriction enzymes SacI and NotI to prepare a DNA fragment containing a full-length protein coding region. This fragment was linked to pYCGPYNot treated with restriction enzymes SacI and NotI to construct a nonScBAT2 high expression vector, nonScBAT2/pYCGPYNot. pYCGPYNot is an yeast expression vector of an YCp type, and the introduced gene is highly expressed with a promoter of pyruvate kinase gene PYK1. Geneticin-resistant gene G418^{r}. as a selective marker for yeast and ampicillin-resistant gene Amp^{r} as a selective marker for *E.coli* were also included.

The high expression vector prepared by the method above was used to transform *Saccharomyces pastorianus* Weihenstephan 34/70 strain according to the method described in Japanese Laid-Open Patent Application No.07-303475. The transformants were selected in a YPD plate medium (1% yeast extract, 2% polypeptone, 2% glucose, 2% agar) containing 300mg/L geneticin.

### Example 12: Analysis of amounts of amyl alcohol isobutanol and isoamyl acetate produced upon beer fermentation test

Fermentation test using a parent strain and the nonScBAT2 highly expressed strain obtained in Example 11 were conducted under the following conditions.

| | |
|---|---|
| Wort extract concentration | 12% |
| Wort content | 1L |
| Wort dissolved oxygen concentration | 10ppm |
| Fermentation temperature | 115°C |
| Yeast pitching rate | 5g/L |

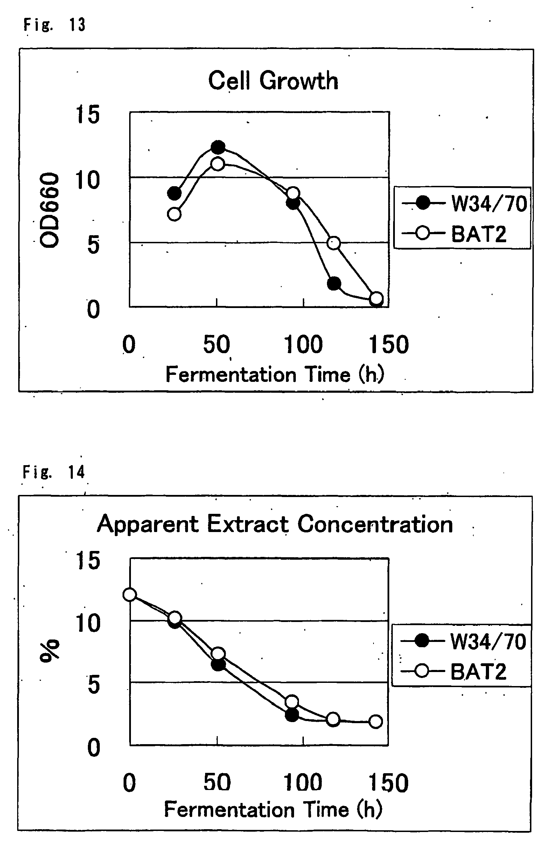

The fermentation broth was sampled with time to observe changes in the amounts of yeast growth (OD660) (Figure 13) and the amounts of extract consumption (Figure 14) with time. Quantifications of higher alcohol and ester concentrations at the end of fermentation were conducted using head-space gas chromatography (J. Am. Soc. Brew. Chem.49:152-157,1991).

Referring to Table 4, the amounts of isobutanol production at the end of fermentation were 11.1ppm in the parent strain and 29.4ppm in the nonScBAT2 highly expressed strain. The amounts of amyl alcohol production were 61ppm in the parent strain and 103.9ppm in the nonScBAT2 highly expressed strain. The amounts of isoamyl acetate production were 1.8ppm in the parent strain and 3.6ppm in the nonScBAT2 highly expressed strain. From these results, it was revealed that the amounts of isobutanol, amyl alcohol and isoamyl acetate produced were increased by about 1.7 to 2.7 times by nonScBAT2 high expression. In these cases, differences in growth rates and extract consumption rates were little between the parent strain and the highly expressed strain.

**Table 4**

| | parent strain (W34/70) | nonScBAT2 highly expressed strain | |
|---|---|---|---|
| Isobutanol | 11.1 | 29.4. | (265%) |
| Amyl alcohol | 61 | 103.9 | (170%) |
| Isoamyl acetate | 1.8 | 3.6 | (200%) |

| | | | |
|---|---|---|---|
| Unit: ppm. Values in parentheses indicate percentages relative to the parent strain. | | | |

As can be appreciated from the above results, by controlling the expression level of the branched-chain amino acid aminotransferase unique to beer yeast disclosed herein, the capacity oft yeast to produce higher alcohol and ester thereof can be controlled As a result, the amounts of amyl alcohol, isobutanol and isoamyl acetate produced can be controlled according to the intended purpose without altering the fermentation procedure or time. Thus, alcoholic drinks with enhanced flavor can be produced.

### INDUSTRIAL APPLICABILITY

According to a method for producing alcoholic drinks of the invention, higher alcohol or ester content that gives rich flavor to the product can be controlled to produce alcoholic drinks with enhanced flavor. Moreover, for malt drinks such as beer where excessive quantity of higher alcohol or ester is disfavored, alcoholic drinks with improved flavor can be produced with the content of higher alcohol or ester being decreased.

## Claims

1. A polynucleotide selected from the group consisting of the following polynucleotides (a) to (d):
(a) as polynucleotide comprising a polynucleotide consisting of the nucleotide sequence represented by SEQ ID NO:1 or SEQ ID NO:3;
(b) a polynucleotide comprising a polynucleotide coding for a protein consisting of the amino acid sequence represented by SEQ ID NO:2 or SEQ ID NO:4;
(c) a polynucleotide comprising a polynucleotide coding for a protein consisting of the amino acid sequence represented by SEQ ID NO:2 or SEQ ID NO:4 with one to 18 amino acids thereof being deleted, substituted, inserted and/or added, and having a branched-chain amino acid aminotransferase activity; and
(d) a polynucleotide comprising a polynucleotide coding for a protein having an amino acid sequence having 95% or higher identity with the amino acid sequence represented by SEQ ID NO:2 or SEQ ID NO:4, and having a branched-chain amino acid aminotransferase activity.

2. A polynucleotide according to Claim 1, selected from the group consisting of the following polynucleotides (g) and (h):
(g) a polynucleotide comprising a polynucleotide coding for a protein consisting of the amino acid sequence represented by SEQ ID NO:2 or SEQ ID NO:4 or of an amino acid sequence represented by SEQ ID NO:2 or SEQ ID NO:4 with 1 to 5 amino acids thereof being deleted, substituted, inserted and/or added, and having a branched-chain amino acid aminotransferase activity; and
(h) a polynucleotide comprising a polynucleotide coding for a protein of an amino acid sequence having 99% or higher identity with the amino acid sequence represented by SEQ ID NO:2 or SEQ ID NO:4, and having a branched-chain amino acid aminotransferase activity.

3. A polynucleotide according to Claim 1, comprising a polynucleotide consisting of the nucleotide sequence represented by SEQ ID NO:1 or SEQ ID NO:3.

4. A polynucleotide according to Claim 1, comprising a polynucleotide coding for a protein consisting of the amino acid sequence represented by SEQ ID NO:2 or SEQ ID NO:4.

5. A polynucleotide according to any one of Claims 1 to 4, wherein the polynucleotide is DNA.

6. A protein coded by the polynucleotide according to any one of Claims 1 to 5.

7. A vector comprising the polynucleotide according to any one of Claims 1 to 5.

8. A yeast introduced with the vector according to Claim 7.

9. A yeast according to Claim 8, having an enhanced capacity of producing amyl alcohol and/or isobutanol and/or isoamyl acetate by being introduced with the vector according to Claim 7.

10. A yeast in which the expression of the polynucleotide (DNA) according to Claim 5 is repressed by disrupting the gene related to the polynucleotide (DNA) according to Claim 5.

11. A yeast according to Claim 9, having an enhanced capacity of producing amyl alcohol and/or isobutanol and/or isoamyl acetate by increasing the expression level of the protein according to Claim 6.

12. A method for producing an alcoholic drink comprising using the yeast according to any one of Claims 8 to 11.

13. A method for producing an alcoholic drink according to Claim 12, wherein the brewed alcoholic drink is a malt drink.

14. A method for producing an alcoholic drink according to Claim 12, wherein the brewed alcoholic drink is wine.

15. A method for assessing a test yeast for its capacity of producing amyl alcohol and/or isobutanol and/or isoamyl acetate, comprising using a primer or a probe comprising at least 15 nucleotides of a nucleotide sequence of a branched-chain amino acid aminotransferase gene having the nucleotide sequence represented by SEQ ID NO:1 or SEQ ID NO:3.

16. A method for assessing a test yeast for its capacity of producing amyl alcohol and/or isobutanol and/or isoamyl acetate, comprising: culturing a test yeast; and determining an expression level of a branched-chain amino acid aminotransferase gene having the nucleotide sequence represented by SEQ ID NO:1 or SEQ ID NO:3.

17. A method for selecting a yeast, comprising: culturing test yeasts; quantifying the protein according to Claim 6 or determining an expression level of a branched-chain amino acid aminotransferase gene having the nucleotide sequence represented by SEQ ID NO:1 or SEQ ID NO:3; and selecting a test yeast having said protein amount or said gene expression level according to the capacity of producing target amyl alcohol and/or isobutanol and/or isoamyl acetate.

18. A method for selecting a yeast according to Claim 17, comprising: culturing a reference yeast and test yeasts; determining expression level of a branched-chain amino acid aminotransferase gene having the nucleotide sequence represented by SEQ ID NO:1 or SEQ ID NO:3 in each yeast; and selecting a test yeast having the gene expressed higher or lower than that in the reference yeast.

19. A method for selecting a yeast according to Claim 17, comprising: culturing a reference yeast and test yeasts; quantifying the protein according to Claim 6 in each yeast; and selecting a test yeast having said protein for a larger or smaller amount than that in the reference yeast.

20. A method for producing an alcoholic drink comprising: conducting fermentation for producing an alcoholic drink using the yeast according to any one of Claims 8 to 11 or an yeast selected by the method according to any one of Claims 17 to 19; and adjusting the production amount of amyl alcohol and/or isobutanol and/or isoamyl acetate.

## Patentansprüche

1. Polynucleotid, ausgewählt aus der Gruppe bestehend aus den folgenden Polynucleotiden (a) bis (d):
(a) ein Polynucleotid umfassend ein Polynucleotid bestehend aus der Nucleotidsequenz dargestellt durch SEQ ID NO:1 oder SEQ ID NO:3;
(b) ein Polynucleotid umfassend ein Polynucleotid, das ein Protein codiert, das aus der Aminosäuresequenz dargestellt durch SEQ ID NO:2 oder SEQ ID NO:4 besteht;
(c) ein Polynucleotid umfassend ein Polynucleotid, das ein Protein codiert, das aus der Amionsäuresequenz dargestellt durch SEQ ID NO:2 oder SEQ ID NO:4 besteht, wobei eine bis 18 Aminosäuren davon entfernt, substituiert, eingefugt, und/oder hinzugefügt sind, und wobei das Protein eine verzweigtkettige Aminosäure-Aminotransferase-Aktivität hat; und
(d) ein Polynucleotid umfassend ein Polynucleotid, das ein Protein codiert, das eine Aminosäuresequenz hat, die eine 95%ige oder höhere Identität mit der Aminosäuresequenz dargestellt durch SEQ ID NO:2 oder SEQ ID NO:4 hat, und wobei das Protein eine verzweigtkettige Aminosäure-Aminotransferase-Aktivität hat.

2. Polynucleotid gemäß Anspruch 1, ausgewählt aus der Gruppe bestehend aus den folgenden Polynucleotiden (g) und (h):
(g) ein Polynucleotid umfassend ein Polynucleotid, das ein Protein codiert, das aus der Aminosäuresequenz dargestellt durch SEQ ID NO:2 oder SEQ ID NO:4 besteht, oder aus einer Aminosäuresequenz dargestellt durch SEQ ID NO:2 oder SEQ ID NO:4 besteht, wobei 1 bis 5 Aminosäuren davon entfernt, substituiert, eingefügt und/oder hinzugefügt sind, und wobei das Protein eine verzweigtkettige Aminosäure-Aminotransferase-Aktivität hat; und
(h) ein Polynucleotid, umfassend ein Polynucleotid, das ein Protein codiert, das eine Aminosäuresequenz hat, die eine 99%-ige oder höhere Identität mit der Aminosäuresequenz dargestellt durch SEQ ID NO:2 oder SEQ ID NO:4 hat, und wobei das Protein eine verzweigtkettige Aminosäure-Aminotransferase-Aktivität hat.

3. Polynucleotid gemäß Anspruch 1, umfassend ein Polynucleotid, das aus der Nucleotidsequenz dargestellt durch SEQ ID NO:1 oder SEQ ID NO:3 besteht.

4. Polynucleotid gemäß Anspruch 1, umfassend ein Polynucleotid, das ein Protein codiert, das aus der Aminosäuresequenz dargestellt durch SEQ ID NO:2 oder SEQ ID NO:4 besteht.

5. Polynucleotid gemäß einem der Ansprüche 1 bis 4, wobei das Polynucleotid DNA ist.

6. Protein codiert durch das Polynucleotid gemäß einem der Ansprüche 1 bis 5.

7. Vektor umfassend das Polynucleotid gemäß einem der Ansprüche 1 bis 5.

8. Hefe, in die der Vektor gemäß Anspruch 7 eingeführt wurde.

9. Hefe gemäß Anspruch 8, die durch die Einführung des Vektors gemäß Anspruch 7 eine gesteigerte Fähigkeit hat, Amylalkohol und/oder Isobutanol und/oder Isoamylacetat herzustellen.

10. Hefe, in der die Expression des Polynucleotids (DNA) gemäß Anspruch 5 durch die Unterbrechung des Gens, das sich auf das Polynucleotid (DNA) gemäß Anspruch 5 bezieht, unterdrückt ist.

11. Hefe gemäß Anspruch 9, die durch den Anstieg der Expressionsspiegels des Proteins gemäß Anspruch 6 eine gesteigerte Fähigkeit zur Herstellung von Amylalkohol und/oder Isobutanol und/oder Isoamylacetat hat.

12. Verfahren zur Herstellung eines alkoholischen Getränks umfassend die Verwendung der Hefe gemäß einem der Ansprüche 8 bis 11.

13. Verfahren zur Herstellung eines alkoholischen Getränks gemäß Anspruch 12, wobei das gebraute alkoholische Getränk ein Malzgetränk ist.

14. Verfahren zur Herstellung eines alkoholischen Getränks gemäß Anspruch 12, wobei das gebraute alkoholische Getränk Wein ist.

15. Verfahren zur Bewertung der Fähigkeit einer Testhefe, Amylalkohol und/oder Isobutanol und/oder Isoamylacetat herzustellen, umfassend die Verwendung eines Primers oder einer Sonde, der/die mindestens 15 Nucleotide einer Nucleotidsequenz eines verzweigtkettigen Aminosäure-Aminotransferasegens umfasst, das die Nucleotidsequenz dargestellt durch SEQ ID NO:1 1 oder SEQ ID NO:3 hat.

16. Verfahren zur Bewertung der Fähigkeit einer Testhefe, Amylalkohol und/oder Isobutanol und/oder Isoamylacetat herzustellen, umfassend: Züchten einer Testhefe; und Bestimmen eines Expressionsspiegels eines verzweigtkettigen Aminosäure-Aminotransferasegens, das die Nucleotidsequenz dargestellt durch SEQ ID NO:1 oder SEQ ID NO:3 hat.

17. Verfahren zum Auswählen einer Hefe, umfassend: Züchten von Testhefen, Quantifizieren des Proteins gemäß Anspruch 6 oder Bestimmen eines Expressionsspiegels eines verzweigtkettigen Aminosäure-Aminotransferasegens, das die Nucleotidsequenz dargestellt durch SEQ ID NO:1 oder SEQ ID NO:3 hat; und Auswählen einer Testhefe, die diese Proteinmenge oder diesen Genexpressionsspiegel hat, entsprechend der Fähigkeit, den Ziel-Amylalkohol und/oder Ziel-Isobutanol und/oder Ziel-Isoamylacetat herzustellen.

18. Verfahren zum Auswählen einer Hefe gemäß Anspruch 17, umfassend: Züchten einer Referenzhefe und von Testhefen; Bestimmen des Expressionsspiegels eines verzweigtkettigen Aminosäure-Aminotransferasegens, das die Nucleotidsequenz dargestellt durch SEQ ID NO:1 oder SEQ ID NO:3 hat, in jeder Hefe; und Auswählen einer Testhefe, die das Gen höher oder niedriger als in der Referenzhefe exprimiert.

19. Verfahren zum Auswählen einer Hefe gemäß Anspruch 17, umfassend: Züchten einer Referenzhefe und von Testhefen; Quantifizieren des Proteins gemäß Anspruch 6 in jeder Hefe; und Auswählen einer Testhefe, die das Protein in einer größeren oder geringeren Menge hat als die Referenzhefe.

20. Verfahren zum Herstellen eines alkoholischen Getränks, umfassend: Durchführen einer Fermentation zur Herstellung eines alkoholischen Getränks unter Verwendung der Hefe gemäß einem der Ansprüche 8 bis 11 oder einer Hefe ausgewählt durch das Verfahren gemäß einem der Ansprüche 17 bis 19; und Anpassen der produzierten Menge von Amylalkohol und/oder Isobutanol und/oder Isoamylacetat.

## Revendications

1. Polynucléotide choisi dans le groupe constitué des polynucléotides (a) à (d) suivants :
(a) un polynucléotide comprenant un polynucléotide constitué de la séquence nucléotidique représentée par SEQ ID NO : 1 ou SEQ ID NO : 3 ;
(b) un polynucléotide comprenant un polynucléotide codant pour une protéine constituée de la séquence d'acides aminés représentée par SEQ ID NO : 2 ou SEQ ID NO : 4 ;
(c) un polynucléotide comprenant un polynucléotide codant pour une protéine constituée de la séquence d'acides aminés représentée par SEQ ID NO : 2 ou SEQ ID NO : 4 avec un à 18 acides aminés de celles-ci étant délétés, substitués, insérés et/ou ajoutés, et possédant une activité aminotransférase pour acides aminés à chaîne ramifiée ; et
(d) un polynucléotide comprenant un polynucléotide codant pour une protéine ayant une séquence d'acides aminés présentant 95 % ou plus d'identité avec la séquence d'acides aminés représentée par SEQ ID NO : 2 ou SEQ ID NO : 4, et possédant une activité aminotransférase pour acides aminés à chaîne ramifiée.

2. Polynucléotide selon la revendication 1, choisi dans le groupe constitué par les polynucléotides (g) et (h) suivants :
(g) un polynucléotide comprenant un polynucléotide codant pour une protéine constituée de la séquence d'acides aminés représentée par SEQ ID NO : 2 ou SEQ ID NO : 4 ou d'une séquence d'acides aminés représentée par SEQ ID NO : 2 ou SEQ ID NO : 4 avec 1 à 5 acides aminés de celles-ci étant délétés, substitués, insérés et/ou ajoutés, et possédant une activité aminotransférase pour acides aminés à chaîne ramifiée ; et
(h) un polynucléotide comprenant un polynucléotide codant pour une protéine constituée d'une séquence d'acides aminés présentant 99 % ou plus d'identité avec la séquence d'acides aminés représentée par SEQ ID NO : 2 ou SEQ ID NO : 4, et possédant une activité aminotransférase pour acides aminés à chaîne ramifiée.

3. Polynucléotide selon la revendication 1, comprenant un polynucléotide constitué de la séquence nucléotidique représentée par SEQ ID NO : 1 ou SEQ ID NO : 3.

4. Polynucléotide selon la revendication 1, comprenant un polynucléotide codant pour une protéine constituée de la séquence d'acides aminés représentée par SEQ ID NO : 2 ou SEQ ID NO : 4.

5. Polynucléotide selon l'une quelconque des revendications 1 à 4, où le polynucléotide est de l'ADN.

6. Protéine codée par le polynucléotide selon l'une quelconque des revendications 1 à 5.

7. Vecteur comprenant le polynucléotide selon l'une quelconque des revendications 1 à 5.

8. Levure dans laquelle le vecteur selon la revendication 7 a été introduit.

9. Levure selon la revendication 8, présentant une capacité amplifiée de production d'alcool amylique et/ou d'isobutanol et/ou d'acétate d'isoamyle par introduction du vecteur selon la revendication 7.

10. Levure dans laquelle l'expression du polynucléotide (ADN) selon la revendication 5 est réprimée par la dissociation du gène lié au polynucléotide (ADN) selon la revendication 5.

11. Levure selon la revendication 9, présentant une capacité amplifiée de production d'alcool amylique et/ou d'isobutanol et/ou d'acétate d'isoamyle par augmentation du taux d'expression de la protéine selon la revendication 6.

12. Procédé de production d'une boisson alcoolique comprenant l'utilisation de la levure selon l'une quelconque des revendications 8 à 11.

13. Procédé de production d'une boisson alcoolique selon la revendication 12, dans lequel la boisson alcoolique brassée est une boisson à base de malt.

14. Procédé de production d'une boisson alcoolique selon la revendication 12, dans lequel la boisson alcoolique brassée est du vin.

15. Procédé permettant d'évaluer une levure à tester pour sa capacité de production d'alcool amylique et/ou d'isobutanol et/ou d'acétate d'isoamyle, comprenant l'utilisation d'une amorce ou d'une sonde comprenant au moins 15 nucléotides d'une séquence nucléotidique d'une gène d'aminotransférase pour acides aminés à chaîne ramifiée ayant la séquence nucléotidique représentée par SEQ ID NO : 1 ou SEQ ID NO : 3.

16. Procédé permettant d'évaluer une levure à tester pour sa capacité de production d'alcool amylique et/ou d'isobutanol et/ou d'acétate d'isoamyle, comprenant : la culture d'une levure à tester; et la détermination d'un taux d'expression d'un gène d'aminotransférase pour acides aminés à chaîne ramifiée ayant la séquence nucléotidique représentée par SEQ ID NO : 1 ou SEQ ID NO : 3.

17. Procédé de sélection d'une levure, comprenant : la culture des levures à tester ; la quantification de la protéine selon la revendication 6 ou la détermination d'un taux d'expression d'un gène d'aminotransférase pour acides aminés à chaîne ramifiée ayant la séquence nucléotidique représentée par SEQ ID NO : 1 ou SEQ ID NO : 3 ; et la sélection d'une levure à tester présentant ladite quantité de protéine ou ledit taux d'expression du gène selon la capacité de production d'alcool amylique et/ou d'isobutanol et/ou d'acétate d'isoamyle cible.

18. Procédé de sélection d'une levure selon la revendication 17, comprenant: la culture d'une levure de référence et des levures à tester ; la détermination du taux d'expression d'un gène d'aminotransférase pour acides aminés à chaîne ramifiée ayant la séquence nucléotidique représentée par SEQ ID NO : 1 ou SEQ ID NO : 3 dans chaque levure ; et la sélection d'une levure à tester ayant le gène exprimé plus ou moins que celui dans la levure de référence.

19. Procédé de sélection d'une levure selon la revendication 17, comprenant: la culture d'une levure de référence et des levures à tester ; la quantification de la protéine selon la revendication 6 dans chaque levure ; et la sélection d'une levure à tester ayant ladite protéine dans une quantité supérieure ou inférieure à celle dans la levure de référence.

20. Procédé de production d'une boisson alcoolique comprenant : la conduction d'une fermentation pour produire une boisson alcoolique en utilisant la levure selon l'une quelconque des revendications 8 à 11 ou une levure sélectionnée par le procédé selon l'une quelconque des revendications 17 à 19 ; et l'ajustement de la quantité de production d'alcool amylique et/ou d'isobutanol et/ou d'acétate d'isoamyle.
